# EUROPÄISCHE PATENTANMELDUNG

(11) **EP 2 662 604 A2**
(43) Veröffentlichungstag der Anmeldung: **13.11.2013**
(21) Anmeldenummer: 13166586.1
(22) Anmeldetag: 06.05.2013
(51) Int. Cl.: F16L 21/03

(54) **Winkelverbinder zum Verbinden eines Fluidsensorstutzens eines Fluidsensors mit einer Fluidleitung**

(30) Priorität: 11.05.2012 DE 102012207892
(71) Anmelder: VERITAS AG, 63571 Gelnhausen (DE)
(72) Erfinder: Sachs, Elmar, 63637 Jossgrund (DE); Wilhelmi, Oliver, 63589 Linsengericht (DE)
(74) Vertreter: Klinski, Robert

(57) **Zusammenfassung**

Die vorliegende Erfindung betrifft einen Winkelverbinder 100 zum Verbinden eines Fluidsensorstutzens 101 eines Fluidsensors 201 mit einer Fluidleitung 203, mit einem Leitungsanschluss 103 zum Anschließen der Fluidleitung 203 und einer Anschlusshülse 105 zur formschlüssigen Aufnahme des Fluidsensorstutzens 101.

## Beschreibung

Die vorliegende Erfindung betrifft einen Winkelverbinder zum Verbinden eines Fluidsensorstutzens sowie ein Fluidsensorsystem mit einem Winkelverbinder.

In Systemen mit strömenden Fluiden wie beispielsweise Abgasen in einem Fahrzeugverbrennungsmotor ist die Erfassung einer Fluideigenschaft, beispielsweise eines Fluiddrucks oder einer Fluidzusammensetzung oft von großer Bedeutung. Hierzu ist es jedoch notwendig, einen Fluidsensor mit einer Fluid führenden Leitung zu verbinden. Dies kann insbesondere in Fahrzeugen problematisch sein, weil bestehende Verbindungsmöglichkeiten zum Verbinden eines Fluidsensorstutzens innerhalb eines Fahrzeugs oftmals sperrig und aufgrund einer vorgegebenen Leitungsführung nur schlecht zu montieren und einzusetzen sind.

Die Druckschrift DE10 2009 039 983 A1 betrifft ein Verbindungselement für Strömungsfluide, bestehend aus einem Verbinderteil mit mindestens zwei Anschlussabschnitten jeweils zum Anschluss an eine Fluidleitung. Dieses Verbindungselement stellt jedoch keine kompakte Anschlussmöglichkeit für einen Fluidsensor bereit.

Es ist die Aufgabe der vorliegenden Erfindung eine kompakte Anschlussmöglichkeit für einen Fluidsensor bereitzustellen.

Diese Aufgabe wird durch Gegenstände mit den Merkmalen nach den unabhängigen Ansprüchen gelöst. Vorteilhafte Ausführungsformen sind Gegenstand der abhängigen Ansprüche, der Beschreibung und der Figuren.

Die vorliegende Erfindung beruht auf der Erkenntnis, dass die obige Aufgabe durch einen abgewinkelten Verbinder, welcher ein Sensorelement, beispielsweise einen Sensorstutzen, unmittelbar mit einer Fluid führenden Leitung verbindet, gelöst werden kann.

Gemäß einem Aspekt betrifft die Erfindung einen Winkelverbinder zum Verbinden eines Fluidsensorstutzens eines Fluidsensors mit einer Fluidleitung, mit einem Leitungsanschluss zum Anschließen der Fluidleitung und einer Anschlusshülse zur formschlüssigen Aufnahme des Fluidsensorstutzens. Dadurch wird beispielsweise der technische Vorteil erreicht, dass der Winkelverbinder bei Leitungsverläufen mit wenig Bauraum verwendet werden kann, nahezu werkstoffunabhängige Verbindungsmöglichkeiten vorhanden sind und eine Dichtwirkung bei verschiedenster Geometrie erreicht wird. Das Fluid kann Gas oder eine Flüssigkeit sein.

In einer vorteilhaften Ausführungsform des Winkelverbinders sind eine Längsachse des Leitungsanschlusses und eine Längsachse der Anschlusshülse zueinander abgewinkelt ausgerichtet. Dadurch wird beispielsweise der technische Vorteil erreicht, dass der Winkelverbinder eine besonders kompakte Form aufweist.

In einer weiteren vorteilhaften Ausführungsform des Winkelverbinders liegt ein Winkel zwischen der Längsachse des Leitungsanschlusses und der Längsachse der Anschlusshülse in einem Winkelbereich zwischen 80° und 100°. Dadurch wird beispielsweise der technische Vorteil erreicht, dass ein seitlicher Anschluss der Fluidleitung an den Fluidsensor vorgenommen werden kann.

In einer weiteren vorteilhaften Ausführungsform des Winkelverbinders weisen der Leitungsanschluss und die Anschlusshülse jeweils einen Fluidinnenkanal auf, welche miteinander strömungstechnisch verbunden sind. Dadurch wird beispielsweise der technische Vorteil erreicht, dass der Fluidsensor eine Eigenschaft des Fluids im Inneren des Winkelverbinders messen kann und die Bauweise des Winkelverbinders kompakt gehalten wird.

In einer weiteren vorteilhaften Ausführungsform des Winkelverbinders sind der Leitungsanschluss und die Anschlusshülse einstückig aus einem formstabilen Material gebildet. Dadurch wird der technische Vorteil erreicht, dass sich die Stabilität und Bruchfestigkeit des Winkelverbinders erhöht.

In einer weiteren vorteilhaften Ausführungsform des Winkelverbinders ist die Anschlusshülse ausgebildet, den Fluidsensorstutzen mittels einer formschlüssigen Rastverbindung zu halten. Dadurch wird der technische Vorteil erreicht, dass eine Montage des Winkelverbinders vereinfacht wird.

In einer weiteren vorteilhaften Ausführungsform des Winkelverbinders umfasst eine Innenwandung der Anschlusshülse zumindest einen Vorsprung oder eine Ausnehmung für die formschlüssige Aufnahme des Fluidsensorstutzens. Dadurch wird der technische Vorteil erreicht, dass sich eine Dichtigkeit einer Verbindung erhöht.

In einer weiteren vorteilhaften Ausführungsform des Winkelverbinders ist in der Anschlusshülse ein Formteil für die formschlüssige Aufnahme des Fluidsensorstutzens angeordnet. Dadurch wird der technische Vorteil erreicht, dass sich die Dichtigkeit der Verbindung erhöht.

In einer weiteren vorteilhaften Ausführungsform des Winkelverbinders umfasst das Formteil einen Vorsprung oder eine Ausnehmung für die formschlüssige Aufnahme des Fluidsensorstutzens. Dadurch wird der technische Vorteil erreicht, dass ein unbeabsichtigtes Lösen des Winkelverbinders verhindert wird.

In einer weiteren vorteilhaften Ausführungsform des Winkelverbinders ist das Formteil herausnehmbar oder mit einer Hülseninnenwandung fest verbunden. Dadurch wird der technische Vorteil erreicht, dass sich sowohl eine Zugfestigkeit als auch eine Dichtigkeit einer Verbindung erhöht.

In einer weiteren vorteilhaften Ausführungsform des Winkelverbinders ist das Formteil aus elastischem Kunststoff, insbesondere aus einem Elastomer-Kunststoff, gebildet. Dadurch wird der technische Vorteil erreicht, dass eine besonders formschlüssige Verbindung hergestellt wird und sich die Dichtigkeit weiter verbessert.

In einer weiteren vorteilhaften Ausführungsform des Winkelverbinders umfasst der Leitungsanschluss zumindest eine Halterippe zum Halten der Fluidleitung. Dadurch wird der technische Vorteil erreicht, dass ein Heruntergleiten der Fluidleitung von dem Winkelverbinder verhindert wird.

In einer weiteren vorteilhaften Ausführungsform des Winkelverbinders ist der Leitungsanschluss in einen Fluidinnenkanal der Fluidleitung einführbar. Dadurch wird der technische Vorteil erreicht, dass der Leitungsanschluss vor Beschädigungen geschützt wird.

In einer weiteren vorteilhaften Ausführungsform des Winkelverbinders umfasst die Anschlusshülse einen Dichtring. Dadurch wird beispielsweise der technische Vorteil erreicht, dass die Dichtigkeit der Verbindung mit einfachen Mitteln erhöht werden kann.

In einer weiteren vorteilhaften Ausführungsform des Winkelverbinders umfasst das Formteil zumindest zwei umlaufende Rippen zum Abdichten des Formteils gegenüber der Anschlusshülse. Dadurch wird beispielsweise der technische Vorteil erreicht, dass eine verbesserte Dichtfunktion erreicht wird.

Gemäß einem weiteren Aspekt wird die erfindungsgemäße Aufgabe durch ein Fluidsensorsystem gelöst, mit einem Winkelverbinder gemäß den oben beschriebenen Ausführungsformen, einem Fluidsensor mit einem Fluidsensorstutzen, welcher in der Anschlusshülse angeordnet und mit der Anschlusshülse formschlüssig verbunden ist; und einer Fluidleitung, welche mit dem Leitungsanschluss verbunden ist. Dadurch werden die gleichen Vorteile erreicht wie durch den oben beschriebenen Winkelverbinder.

Ausführungsbeispiele der Erfindung sind in der Zeichnung dargestellt und werden im Folgenden näher beschrieben.

Es zeigen:
- Fig. 1: eine Querschnittsansicht durch eine erste Ausführungsform eines Winkelverbinder;
- Fig. 2: eine Querschnittsansicht durch eine zweite Ausführungsform eines Winkelverbinder; und
- Fig. 3: eine perspektivische Ansicht eines Fluidsensorsystems.

In Fahrzeugen moderner Bauart wird eine Vielzahl unterschiedlicher Sensoren verwendet. Ein Teil dieser Sensoren dient als Fluidsensor zum Messen von Eigenschaften eines Fluids, wie beispielsweise Gas oder Flüssigkeit. Ein derartiger Fluidsensor kann beispielsweise eine Temperatur, einen Druck, eine Dichte, eine Zusammensetzung, eine Strömungsgeschwindigkeit, eine Feuchtigkeit oder eine andere physikalische Eigenschaft des zu untersuchenden Fluids bestimmen. Zu diesem Zweck weist der Fluidsensor in der Regel einen Fluidstutzen auf, welcher beispielsweise ein Messkopf oder ein Ankopplungsstück für die Fluidweiterleitung zu einem Messkopf sein kann.

Das Fluid wird in einer Fluidleitung geführt. Bei dieser Fluidleitung handelt es sich beispielsweise um einen faserverstärkten Gummischlauch, in dessen Inneren das Fluid transportiert wird. In einer anderen Ausführungsform wird die Fluidleitung durch ein Kunststoffrohr gebildet. Im Allgemeinen können als Fluidleitung alle Vorrichtungen angesehen werden, die geeignet sind, im Inneren ein Fluid zu führen oder zu transportieren.

Um die gewünschte Eigenschaft des Fluids zu messen, wird der Fluidsensor in Kontakt mit dem Fluid gebracht. Dazu wird der Fluidsensor an die Fluidleitung angeschlossen. Herkömmliche Verbinder für diese Aufgabe sind sperrig und nur schlecht an die räumlichen Gegebenheiten im Inneren eines Fahrzeugs angepasst. Der erfindungsgemäße Winkelverbinder löst dieses Problem und ermöglicht eine Reduzierung des benötigten Bauraums bei gleichzeitig komplexer Leitungsführung.

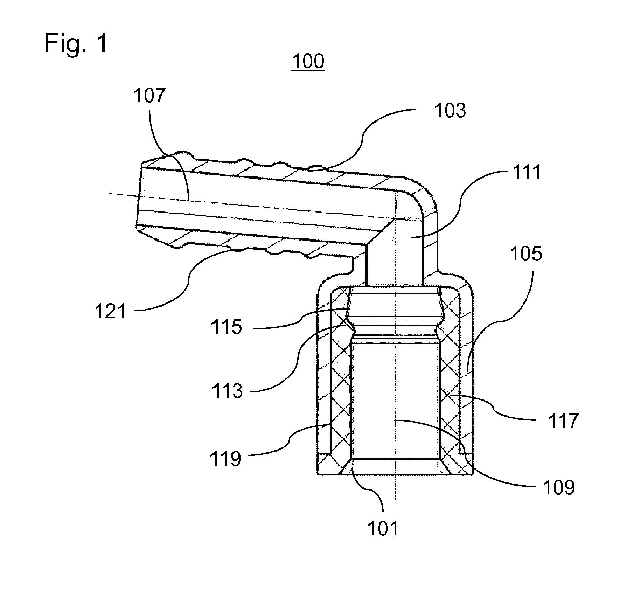

Fig. 1 zeigt eine Querschnittsansicht durch eine erste Ausführungsform eines Winkelverbinders 100. Der Winkelverbinder 100 umfasst einen Leitungsanschluss 103 zum Anschließen einer Fluidleitung und eine Anschlusshülse 105 zur formschlüssigen Aufnahme des gestrichelt dargestellten Fluidsensorstutzens 101. Der Fluidsensorstutzen 101 ist in die Anschlusshülse 105 einführbar und der Winkelverbinder 100 wird mit der Anschlusshülse 105 auf den Fluidsensorstutzens 101 gesteckt.

Die Anschlusshülse 105 ist um eine Achse 109 herum rotationssymmetrisch aufgebaut. Der Leitungsanschluss 103 dient zum Anschließen und Verbinden mit einer Fluidleitung, die das Fluid oder das Fluid an den Fluidsensorstutzen 101 führt. Beispielsweise kann der Leitungsanschluss 103 derart ausgestaltet sein, dass ein Schlauch als Fluidleitung auf den Leitungsanschluss aufsteckbar ist. Der Leitungsanschluss 103 ist um die Achse 107 herum symmetrisch aufgebaut und weist eine Halterippe 121 auf, die beispielsweise ein Herabrutschen des Schlauches verhindert. Das Fluid wird in dem Winkelverbinder in einem Fluidinnenkanal 111 geführt

Der Fluidsensor ist beispielsweise in einem Abgassystem, einem Lüftungssystem, einem Kraftstoffsystem, einem Öl- oder Schmiersystem, einem Kühlmittelsystem, einem Bremssystem oder einem anderen System angeordnet, in dem ein Fluid verwendet wird. Im Inneren der Anschlusshülse 105 ist ein Formteil 117 angeordnet, das beispielsweise aus einem elastischen Material besteht. Die Anschlusshülse 105 und das Formteil 117 sind beispielsweise an einer Berührungsfläche fest miteinander verbunden, wie beispielsweise verklebt.

An der Hülseninnenwandung 119 ist ein umlaufender Vorsprung 113 oder eine umlaufende Ausnehmung 115 gebildet, die in eine entsprechende Form in dem Fluidsensorstutzen 101 eingreifen und dadurch die Dichtwirkung des Winkelverbinders 100 verbessert. Darüber hinaus wird durch diese Maßnahme erreicht, dass ein unbeabsichtigtes Lösen der mechanischen Verbindung zwischen Fluidsensorstutzen 101 und Anschlusshülse 105 verhindert wird. In einer weiteren Ausführungsform umfasst die Anschlusshülse einen Dichtring. Die dichtende Wand des Formteils 117 kann an jede Kontur des Fluidsensorstutzens angepasst werden. Mit einer Hinterschneidung kann eine Verbindung druckdicht ausgebildet sein, beispielsweise bis zu 6 bar.

In einer weiteren Ausführungsform des Winkelverbinders 100 kann auf ein in der Anschlusshülse 105 liegendes Formteil 117 verzichtet werden. In diesem Fall kann die Anschlusshülse 105 eine formschlüssige Ausgestaltung aufweisen.

Der Winkelverbinder 100, der Leitungsanschluss 103, die Anschlusshülse 105 oder das Formteil 117 können aus gasdichtem Material, wie beispielsweise Kunststoff hergestellt sein. Geeignete Kunststoffe sind beispielsweise Polyamid, Polybutadien, Polyvinylchlorid, Polypropylen oder Polyethylen.

In einer alternativen Ausführungsform kann der Winkelverbinder 100, der Leitungsanschluss 103, die Anschlusshülse 105 oder das Formteil 117 beispielsweise aus Fluorkautschuk-Material (FKM-Material) hergestellt sein. Das Fluorkautschuk-Material bezeichnet eine ganze Gruppe von Kautschuken, die als gemeinsames Merkmal Vinyliden(di)fluorid (VDF) als eines ihrer Monomere besitzen. In einer besonderen Ausführungsform ist das Fluorkautschuk-Material ein peroxidisch vernetztes Fluorelastomer, das mit Ruß gefüllt ist und einen Fluorgehalt von ca. 68% hat. Dieses Material ist besonders geeignet, da es eine hervorragende Kälteflexibilität aufweist und der Schmelzpunkt bei ca. -20°C liegt.

Im Allgemeinen ist das Herstellungsmaterial nicht auf ein bestimmtes Material beschränkt und es können unterschiedliche Materialien zur Herstellung der einzelnen Komponenten verwendet werden. Im Allgemeinen ist es vorteilhaft, unterschiedliche Materialien für die einzelnen Bauteile zu verwenden und deren vorteilhafte Eigenschaften zu kombinieren. Das Material der Anschlusshülle des Winkelverbinders kann aufgrund der mechanisch erfolgenden Dichtwirkung beliebig gewählt werden.

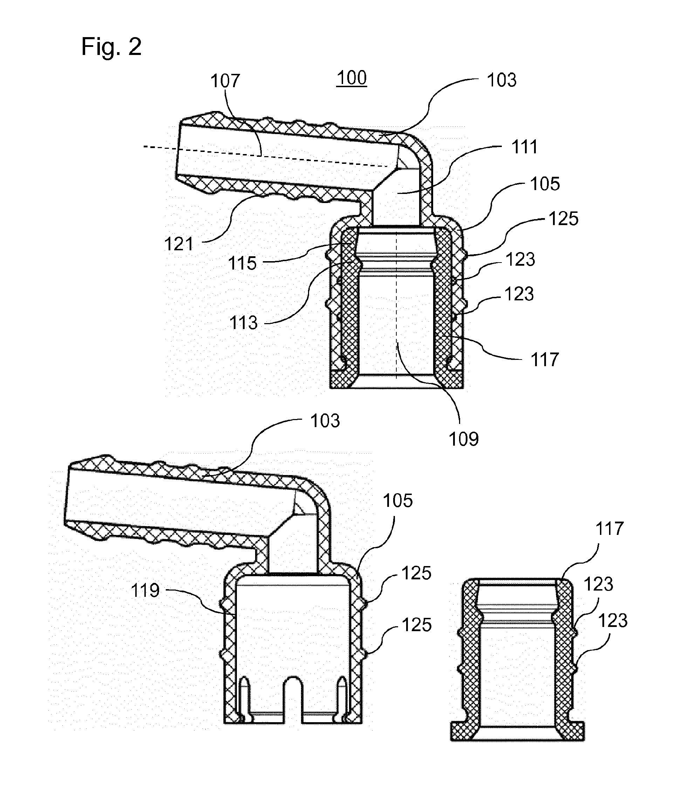

Fig. 2 zeigt eine Querschnittsansicht durch eine zweite Ausführungsform eines Winkelverbinders 100. In dieser Ausführungsform wird die Anschlusshülse 105 mit dem Formteil 117 verrastet. Das Formteil 117 umfasst zwei umlaufende Rippen 123. Die Rippen 123 ragen auf der Außenseite des Formteils 117 heraus und umlaufen das im Querschnitt runde Formteil 117 in paralleler Weise. Die umlaufenden Rippen 123 erhöhen beim Einsetzen des Fluidsensorstutzens den Druck des Formteils 117 auf die glatte Hülseninnenwandung 119 und wirken als zusätzliches Dichtelement. Durch die umlaufenden Rippen 123 auf der Außenseite des Formteils 117 wird somit eine verbesserte Dichtfunktion erreicht.

Zusätzlich umfasst die Anschlusshülse 105 an der Außenseite zwei parallele umlaufende Verstärkungsrippen 125, die die Anschlusshülse 105 konstruktiv verstärken und ein Greifen der Anschlusshülse 105 erleichtern, wie beispielsweise beim Abziehen des Winkelverbinders 100. Die übrigen Bezugszeichen bezeichnen die gleichen Komponenten wie diejenigen aus Fig. 1.

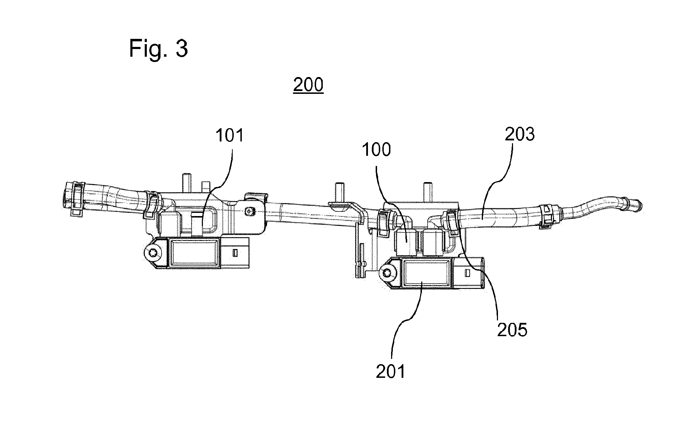

Fig. 3 zeigt eine perspektivische Ansicht eines Fluidsensorsystems 200. Das Fluidsensorsystem umfasst die Fluidsensoren 201 und die Fluidleitungen 203, wie beispielsweise Gummischläuche. Die Fluidleitungen 203 und Fluidsensoren 201 sind mithilfe des Winkelverbinders 100 miteinander verbunden. Zu diesem Zweck wird die Anschlusshülse 105 auf die Fluidsensorstutzen 101 aufgesteckt. Die Fluidleitungen 203 sind auf den Leitungsanschluss 103 aufgesteckt und mit Schellen 205 gesichert.

Durch den erfindungsgemäßen Winkelverbinder 100 wird der benötigte Bauraum im Leitungsverlauf reduziert und eine werkstoffunabhängige Dichtwirkung erzielt. Durch Anpassung der Anschlusshülse oder des Formteils im Inneren der Anschlusshülse wird eine Dichtwirkung erzielt, die unabhängig von der Geometrie des Fluidsensorstutzens ist. Ein Winkelverbinder mit rotationssymmetrischer Anschlusshülse ist gegenüber dem Fluidstutzen drehbar und bis zu hohen Drücken druckdicht.

Im Allgemeinen ist die Form der Anschlusshülse 105 und des Formteils 117 nicht auf die gezeigte Form beschränkt, so dass je nach Fluidsensorstutzen unterschiedlich angepasste Formen verwendet werden können. Insbesondere kann die Anschlusshülse mittels unterschiedlicher Formteile an unterschiedliche Fluidsensorstutzen angepasst werden.

Alle in den Figuren gezeigten und in der Beschreibung erläuterten Einzelmerkmale können in beliebiger sinnvoller Weise miteinander kombiniert werden, um gleichzeitig deren vorteilhafte Wirkungen zu realisieren.

**BEZUGSZEICHENLISTE**

| | |
|---|---|
| 100 | Winkelverbinder |
| 101 | Fluidsensorstutzen |
| 103 | Leitungsanschluss |
| 105 | Anschlusshülse |
| 107 | Längsachse |
| 109 | Längsachse |
| 111 | Fluidinnenkanal |
| 113 | Vorsprung |
| 115 | Ausnehmung |
| 117 | Formteil |
| 119 | Hülseninnenwandung |
| 121 | Halterippe |
| 123 | Rippen |
| 125 | Verstärkungsrippen |
| 200 | Fluidsensorsystem |
| 201 | Fluidsensors |
| 203 | Fluidleitung |
| 205 | Schelle |

## Patentansprüche

1. Winkelverbinder (100) zum Verbinden eines Fluidsensorstutzens (101) eines Fluidsensors (201) mit einer Fluidleitung (203), mit:
einem Leitungsanschluss (103) zum Anschließen der Fluidleitung (203); und
einer Anschlusshülse (105) zur formschlüssigen Aufnahme des Fluidsensorstutzens (101).

2. Winkelverbinder (100) nach Anspruch 1, wobei eine Längsachse (107) des Leitungsanschlusses (103) und eine Längsachse (109) der Anschlusshülse (105) zueinander abgewinkelt ausgerichtet sind.

3. Winkelverbinder (100) nach Anspruch 2, wobei ein Winkel zwischen der Längsachse (107) des Leitungsanschlusses (103) und der Längsachse (109) der Anschlusshülse (105) in einem Winkelbereich zwischen 80° und 100° liegt.

4. Winkelverbinder (100) nach einem der vorstehenden Ansprüche, wobei der Leitungsanschluss (103) und die Anschlusshülse (105) jeweils einen Fluidinnenkanal (111) aufweisen, welche miteinander strömungstechnisch verbunden sind.

5. Winkelverbinder (100) nach einem der vorstehenden Ansprüche, wobei die Anschlusshülse (105) ausgebildet ist, den Fluidsensorstutzen (101) mittels einer formschlüssigen Rastverbindung zu halten.

6. Winkelverbinder (100) nach einem der vorstehenden Ansprüche, wobei eine Innenwandung der Anschlusshülse (105) zumindest einen Vorsprung (113) oder eine Ausnehmung (115) für die formschlüssige Aufnahme des Fluidsensorstutzens (101) umfasst.

7. Winkelverbinder (100) nach einem der vorstehenden Ansprüche 1 bis 6, wobei in der Anschlusshülse (105) ein Formteil (117) für die formschlüssige Aufnahme des Fluidsensorstutzens (101) angeordnet ist.

8. Winkelverbinder (100) nach Anspruch 7, wobei das Formteil (117) einen Vorsprung (113) oder eine Ausnehmung (115) für die formschlüssige Aufnahme des Fluidsensorstutzens (101) umfasst.

9. Winkelverbinder (100) nach Anspruch 7 oder 8, wobei das Formteil (117) herausnehmbar oder mit einer Hülseninnenwandung (119) fest verbunden ist.

10. Winkelverbinder (100) nach einem der Ansprüche 7 bis 9, wobei das Formteil (117) zumindest zwei umlaufende Rippen zum Abdichten des Formteils (117) gegenüber der Anschlusshülse (105) umfasst.

11. Fluidsensorsystem (200), mit:
einem Winkelverbinder (100) gemäß einem der Ansprüche 1 bis 10;
einem Fluidsensor (201) mit einem Fluidsensorstutzen (101), welcher in der Anschlusshülse (105) angeordnet und mit der Anschlusshülse (105) formschlüssig verbunden ist; und
einer Fluidleitung (203), welche mit dem Leitungsanschluss (103) verbunden ist.
